# EUROPEAN PATENT APPLICATION

(11) **EP 3 650 012 A1**
(43) Date of publication of application: **13.05.2020**
(21) Application number: 18829016.7
(22) Date of filing: 04.07.2018
(51) Int. Cl.: A61K 9/16, A61K 9/00, A61K 31/79, A61P 31/12, A61P 31/04, A61K 9/08, A61K 9/14, A61K 33/18

(54) **SOLID COMPOSITION COMPRISING IODINE AGENT AND SODIUM CHLORIDE HAVING IMPROVED WATER SOLUBILITY, AND ANTIVIRAL AND ANTIMICROBIAL COMPOSITION FOR EYE, ORAL CAVITY, NASAL CAVITY OR INHALATION CONTAINING AQUEOUS SOLUTION THEREOF**

(30) Priority: 04.07.2017 KR 20170084892; 08.02.2018 KR 20180015894
(71) Applicant: Fluchem Ltd, Daejeon 34078 (KR)
(72) Inventor: KIM, Dae Whang, Yuseong-gu Daejeon 34083 (KR); PARK, Yang Ok, Yuseong-gu Daejeon 34083 (KR); KIM, Seung Shin, Songpa-gu Seoul 05658 (KR); KIM, Jeung Ouk, Yuseong-gu Daejeon 34082 (KR)
(74) Representative: Adam, Holger
(86) International application number: PCT/KR2018/007601
(87) International publication number: WO 2019/009630

(57) **Abstract**

The present invention relates to a solid composition comprising an iodine agent and sodium chloride having improved water solubility and to an antiviral and antimicrobial composition for an eye, oral cavity, nasal cavity or inhalation containing an aqueous solution thereof, wherein the solid composition comprises an iodine agent and sodium chloride or a mixture of an iodine agent, sodium chloride and a flavoring agent and is excellent in not only storage stability but also water solubility as a dissolution rate thereof for water is 30 seconds or less. In addition, the aqueous solution in which the solid composition is dissolved in water has an excellent effect of shielding from the unpleasant or irritating odor and taste of an iodine agent while also not causing pain or irritation, has an outstanding effect of preventing or treating infectious respiratory diseases such as a cold or influenza, and is applicable to all parts of a respiratory organ, such that the solution can be usefully used for the prevention or treatment of respiratory infectious diseases.

## Description

### Technical Field

The present invention relates to a solid composition having improved water solubility and containing an iodine agent and sodium chloride, and to an antiviral and antibacterial ocular, oral, nasal, or inhalation composition comprising an aqueous solution thereof.

### Background Art

Representative pathogenic viruses causing infectious diseases in the respiratory tract are rhinovirus, adenovirus, coronavirus, and influenza virus, but at least 200 kinds of various viruses are known. Respiratory infectious diseases encompass a wide range of clinical symptoms ranging from relatively light colds to severe illnesses, such as flu, bronchitis, pneumonia, and acute respiratory distress syndrome, and are reported to show the highest morbidity and mortality worldwide.

Respiratory infectious diseases, which are one of the most common diseases causing patients to visit medical institutions even in advanced countries, are known to account for a large part of medical expenses. In general, the symptoms of respiratory infectious diseases are reported to occur 6-8 times a year in children and 2-4 times a year in adults. However, since respiratory infectious diseases express similar symptoms and signs, the causative pathogens thereof are difficult to distinguish by clinical features alone, so antibiotic administration or symptomatic treatment is performed merely for the purpose of alleviating clinical symptoms and preventing secondary bacterial infections without identifying the exact causes of the respiratory infectious diseases at the time of treatment.

Disinfectants are drugs that have a wide range of activity against a large number of pathogens causing infections, and do not cause resistance while destroying bacteria, viruses, and many other microorganisms. In particular, iodine disinfectants among the disinfectants are known to show a wide range of activity against various viruses and bacteria in a short time.

Povidone iodine, which is a chemical blend of polyvinylpyrrolidone (povidone, PVP) and iodine, usually contains 9-12% iodine. Povidone iodine has been widely used as a sterilizing disinfectant for preventing and treating infections of wounds, and is known to be effective against yeasts, molds, fungi, viruses, and protozoans.

Especially, povidone iodine can kill SARS-coronavirus and various avian influenza viruses in a short time, and has been reported to inactivate highly pathogenic H5N1 and low pathogenic H5N3, H7N7, and H9N7 below a detection limit within less than 10 seconds. In addition, povidone iodine has been revealed to have an antiviral effect even against H1N1, H3N2, H1N2 and MERS-coronavirus, and has been reported to have a killing effect on various respiratory infection bacteria (Eggers, M. et al., Infect Dis Ther., 4(4), 491-501, 2015; Ito, H. et al., Dermatology, 212 Suppl 1, 115-118, 2006; Kawana, R. et al., Dermatology, 195 Suppl 2, 29-35, 1997; Rikimaru, T. et al., Dermatology, 204 Suppl 1, 15-20, 2002; Sriwilaijaroen, N. et al., Virol J., 6, 124, 2009).

Most of conventional commercial povidone iodine was prepared in a solution state despite excellent activity and widespread application thereof. A high concentration of 10% povidone iodine solution is relatively stable and thus suitable for long-term storage, but high concentrations of povidone iodine intended to disinfect eyes, nasal cavity, oral cavity, or the lower respiratory tract may damage cilia, and thus cannot be used in mucosal or epithelial layers with cilia, where only povidone iodine with a low concentration of 0.3% or less can be used. However, in a case where a povidone iodine solution is distributed as a low-concentration of dilution solution for convenience of use, the activity of an iodine agent drops rapidly and the lifetime of products is shortened, and thus such a dilution solution needs to be discarded. Therefore, low concentrations of povidone iodine are difficult to distribute or store for a long time, and a disinfectant containing 0.3% or less povidone iodine has not been marketed as a product until the present.

Hence, a high concentration of iodine solution is required to be diluted immediately before use when a low concentration of iodine solution needs to be used, but since diluting a high concentration of iodine solution before use is inconvenient and the concentration of a commercially available high concentration of iodine solution is not constant, an ordinary person or a patient cannot prepare a solution with an accurate concentration to suit for the application just before use.

Solid povidone iodine is very stable while low concentrations of povidone iodine solutions are unstable. Solid povidone iodine, when stored in a sealed state, is very stable not only at room temperature but also at a temperature of 65°C, with an effective iodine loss of about 0.5% for 3 years. Therefore, solid povidone iodine is a stable material without a loss of activity thereof and is storable for a long time, and thus solid povidone iodine can be a good solution to a storage stability problem. However, when practically used in homes or hospitals, solid povidone iodine takes a long time to dissolve in water. Povidone iodine is soluble in water, but povidone iodine takes a long time to completely dissolve in water since povidone iodine agglomerates on the water surface and sticks to the container surface and thus is not easily separated.

US Patent No. 04950653 discloses that it takes 45 minutes to dissolve povidone iodine in water. Povidone iodine, when mixed with urea and sugar alcohol, takes 2 minutes or longer to dissolve in water, but there is a limitation in application of povidone iodine due to the problems of stability and safety, such as urea inclusion. There is no prior art document that discloses a composition, which employs solid povidone iodine for storage stability and is convenient for use during use through improved solubility.

Meanwhile, iodine disinfectants have excellent antiviral and antibacterial effects, but when iodine disinfectants are used in the eyes, nasal cavity, oral cavity, and the lower respiratory tract, the taste, smell, unpleasantness, irritation, and the like by iodine cause a great sense of repulsion in patients. In particular, iodine is a single substance, but encompasses a full range of odors ranging from light to heavy odors as if the iodine was a cocktail of several odorous substances. Therefore, merely the proper mixing of several fragrant ingredients without reflection of these characteristics like in a conventional art makes it difficult to mask a wide range of iodine odors without irradiation or side effects.

In addition, the nasal cavity is a very sensitive organ since the nasal cavity is first exposed to external materials so as to detect extremely low concentrations of odors and has a large distribution of nerves so as to react sensitively to even a small amount of extraneous toxins. Moreover, the irritation is also a serious problem even in a case of the inhalation into the bronchus or lower respiratory tract. Accordingly, there is a need for a method capable of causing irritation while masking an unpleasant taste and smell of iodine.

As for prior art documents, [Yoshitaka, T. et al., Journal of Health Science, 52(3), 324-328, 2006] discloses effects of green tea-based drinks on odor and efficacy of povidone iodine; Korean Patent Publication No. 10-2017-0033925 discloses antiviral and antibacterial spray compositions; US Patent Publication No. 2006-0280809 discloses iodine-based anti-infective compositions for use in ears and nose; US Patent Nos. 06171611 and 06165494 disclose a nasal moisturizing saline and a mouthwash solution each containing iodine; and Chinese Patent No. 001203864 discloses a spray dosage form of povidone iodine. However, there is no prior art document that discloses a composition, which can be administered as one drug to all parts of the respiratory tract while maintaining treatment effects of iodine and can completely mask both the taste and smell of iodine without irritation.

### Detailed Description of the Invention

### Technical Problem

An aspect of the present invention is to provide a solid composition having improved water solubility and containing an iodine agent and sodium chloride and an antiviral and antibacterial ocular, oral, nasal, or inhalation composition containing an aqueous solution of the solid composition.

### Technical Solution

In accordance with an aspect of the present invention, there is provided a solid composition containing an iodine agent and sodium chloride, wherein the solid composition contains a mixture of 0.2-50 parts by weight of the iodine agent and 100 parts by weight of the sodium chloride and has a dissolution rate of 30 seconds or less in water. Preferably, the solid composition contains a mixture of 0.4-25 parts by weight of the iodine agent and 100 parts by weight of the sodium chloride and has a dissolution rate of 15 seconds or less in water.

In addition, when the solid composition having a dissolution rate of 30 seconds or less is dissolved in 100 ml of water, such a solution contains 0.01-0.5 w/v% of an iodine agent and 1-5 w/v% of sodium chloride. In a case of the iodine agent, especially, povidone iodine, the minimum concentration thereof to show a pharmaceutical effect, is 0.01 w/v%. Even a solid composition containing 0.2-50 parts by weight of the iodine agent and 100 parts by weight of sodium chloride may show a low pharmaceutical effect if the final concentration of povidone iodine dissolved in water is less than 0.01 w/v%, and thus such a solid composition is not preferable. In addition, a solid composition causes severe irritation when applied to the respiratory tract if the final concentration of povidone iodine dissolved in water exceeds 0.5 w/v%, and thus such a solid composition is not preferable.

The iodine agent is selected from the group consisting of povidone iodine, cadexomer iodine, and poloxamer iodine, and povidone iodine having excellent antiviral and antibacterial effects is preferably used.

The sodium chloride is a water-soluble solid salt, and 0.2-50 parts by weight of the iodine agent may be mixed with 100 parts by weight of at least one salt, which is selected from salts generated by binding a cation selected from the group consisting of sodium, potassium, ammonium, lithium, or zinc and an anion selected from the group consisting of a chloride, a bromide, or an iodide, but 100 parts by weight of sodium chloride is preferably used.

The solid composition is preferably in a powder form, and the size of powder particles is more preferably 30 mesh or more (600 µm or less), and most preferably 60 mesh or more (250 µm or less).

Furthermore, the solid composition may contain 0.001-2 parts by weight of a fragrant substance. More preferably, the fragrant substance is at least one selected from butyl acetate, amyl acetate, isoamyl acetate, hexyl acetate, 2-hexyl acetate, ethyl propionate, butyl propionate, isobutyl propionate, isoamyl propionate, ethyl butyrate, ethyl 2-methylbutyrate, butyl isobutyrate, ethyl hexanoate, ethyl pivalate, methyl 4-methylvalerate, ethyl valerate, ethyl isovalerate, isopropyl isovalerate, ethyl lactate, 2-pentanone, heptanoic acid, 3-heptanone, 2,3-hexanedione, d-camphor, p-methyl anisole, 2-methoxy-6-methyl pyrazine, 1,8-cineole, 1,4-cineole, benzyl methyl ether, 3-heptanol, isoamyl isovalerate, butyl valerate, butyl lactate, methyl benzoate, ethyl benzoate, propyl benzoate, butyl benzoate, isobutyl benzoate, amyl benzoate, isoamyl benzoate, methyl phenylacetate, ethyl phenylacetate, benzyl acetate, sassafras acetate, isobornyl acetate, ethyl 3-phenylpropionate, 2-methyl-5-ethoxypyrazine, 2-methyl-6-ethoxypyrazine, 2,3-diethyl-5-methylpyrazine, 5-methyl-6,7-dihydro-5H-cyclopentapyrazine, 2-acetyl pyrazine, acetyl pyridine, 2-acetyl pyridine, 3-acetyl pyridine, 2-t-butyl cyclohexanone, p-dimethyl-hydroquinone, isoquinoline, 2,3-dimethyl-benzofuran, 3,6-dimethyl benzofuranone, ambernaphthofuran, p-t-butyl-cyclohexanone, thymol methyl ether, 3-phenylpropyl alcohol, 2,6-dimethoxyphenol, 2-t-butyl-6-methyl-phenol, l-menthol, dl-menthol, d-neomenthol, menthone lactone, p-ethyl acetophenone, skatole, diphenyl ether, β-naphthyl methyl ether, β-naphthyl ethyl ether, saffron indenone, ambroxide, (-)-ambroxide, phenylacetic acid, watermelon ketone, dihydroactinidiolide, 3-butyl-phthalide, borneol, dl-isoborneol, thymol, exaltolide, exaltone, menthyl lactate, methyl dihydrojasmonate, ethylene brassylate, t-hydrofurfuryl phenylacetate, 2-(3-phenylpropyl)pyridine), (±)-muscone, (-)-muscone, isomuscone, normuscone, cyclohexadecanone, celestolide, sandal cyclopropane, aniseed oil, eucalyptus oil, peppermint oil and spearmint oil.

The solid composition containing a mixture of 0.2-50 parts by weight of the iodine agent and 100 parts by weight of the sodium chloride in the present invention does not lose its activity even for a long-term storage and has high storage stability, and it is preferable that such a solid composition is stored in a moisture-proof, light-shielding, and airtight conditions, and then dissolved in water (USP-grade water for injection, distilled water, purified water, physiological saline, tap water, or sterile water) immediately before use. In a case where the solid composition is dissolved in physiological saline immediately before use, the concentration of sodium chloride in the final aqueous solution composition should not exceed 5 w/v%.

In still another aspect, the present invention is directed to an ocular, oral, nasal, or inhalation antiviral and antibacterial composition containing, as an active ingredient, an aqueous solution containing 0.01-0.5 w/v% of an iodine agent and 1-5 w/v% of sodium chloride, the aqueous solution being obtained by dissolving a solid composition containing a mixture of 0.2-50 parts by weight of an iodine agent and 100 parts by weight of sodium chloride, and more preferably, the aqueous solution further contains 0.0001-0.01 w/v% of a fragrant substance. Most preferably, the aqueous solution composition contains 0.01-0.5 w/v% of an iodine agent, 1.2-3.5 w/v% of sodium chloride, and 0.0001-0.01 w/v% of a fragrant substance.

However, among the iodine agent, sodium chloride, and fragrant substance contained in the aqueous solution composition, less than 0.01 w/v% (out of the above w/v% range) of the iodine agent results in low antiviral and antibacterial effects and thus is not preferable, and more than 0.5 w/v% (out of the above w/v% range) of the iodine agent increases the irritation to the oral cavity and respiratory tissue and causes a strong unpleasant or repulsive smell and taste of the iodine agent and thus is not preferable. In the aqueous solution composition, less than 1 w/v% of sodium chloride results in low effects of masking the unpleasant or repulsive smell and taste of the iodine agent and thus is not preferable, and more than 5 w/v% of sodium chloride increases the irritation to the oral cavity and respiratory tissue and thus is not preferable. Less than 0.0001 w/v% of the fragrant substance contained in the aqueous solution composition results in low effects of masking an unpleasant or repulsive smell and taste and thus is not preferable, and a high concentration of the fragrant substance exceeding 0.01 w/v% causes the irritation of the fragrant substance itself to the respiratory tract and cannot be used in the respiratory tract other than the oral cavity and thus is not preferable.

The iodine agent is selected from the group consisting of povidone iodine, cadexomer iodine, and poloxamer iodine, and povidone iodine having excellent antiviral and antibacterial effects is preferably used.

The sodium chloride is a substance having both osmosis and palate stimulation. An osmotic substance can suppress stimulations, such as cooling pain and cough reflex, and a palate-stimulating substance can mask an unpleasant or repulsive smell and taste of an iodine agent. Therefore, the use of sodium chloride like in the present invention can mask both an unpleasant or repulsive smell and taste of an iodine agent without the irritation due to iodine.

The sodium chloride can improve pharmaceutical effects of the composition by freeing pathogens while absorbing water from the epithelial layer of the respiratory tract. The composition may contain at least one salt selected from salts of a cation selected from the group consisting of lithium, sodium, potassium, magnesium, calcium, or zinc and an anion selected from the group consisting of a chloride, a bromide, an iodide, a sulfate, a phosphate, an acetate, a carbonate, a lactate, a succinate, a tartrate, or a citrate, and may also contain a sugar alcohol, such as glycerol, mannitol, sorbitol, xylitol, or maltitol, and an organic osmotic agent, such as acetamide, but sodium chloride is preferably used.

The fragrant substance is used to mask the smells and tastes of iodine. The smells and tastes of iodine can be largely masked by merely mixing sodium chloride with an iodine agent, but the fragrant substance is additionally used to completely mask iodine smell and taste residues. Specifically, the fragrant substance may employ substances selected from the fragrant substances registered by the International Fragrance Association (IFRA), and substances usable as both a fragrant substance and a flavoring agent are preferably used, and stable substances not reacting with iodine are preferably used, and compounds having a functional group, such as saturated alkyl, acid, alcohol, ester, ether, lactone, ketone, or aromatic group, are preferably used.

In particular, the present inventors confirmed that iodine is a single substance and encompasses a full range of odors ranging from light to mild and heavy odors as if the iodine was a cocktail of several odorous substances. The present inventors found that in order to effectively mask such light, middle, and heavy odors, instead of simple mixing of several fragrant ingredients, the light odors, which are first felt by the nose out of the iodine odors, can be masked through fragrant substance group A (fragrant substances with high volatility having a vapor pressure of 1.0-120 mmHg at 25°C) ; the middle odors, which are felt in the mouth out of the iodine odors, can be masked through fragrant substance group B (fragrant substances with middle volatility having a vapor pressure of 0.001-1.0 mmHg at 25°C); and the heavy odors coming out to the last in the neck, out of the iodine odors, can be masked through fragrant substance group C (fragrant substances with low volatility having a vapor pressure of less than 0.001 mmHg at 25°C). Accordingly, the present inventors found that the smell and taste of iodine can be completely masked and the irritation can be lowered through even a tiny amount of the fragrant substance, by using the above fragrant substance group A, fragrant substance group B, and fragrant substance group C in mixture.

The fragrant substance is at least one selected from butyl acetate, amyl acetate, isoamyl acetate, hexyl acetate, 2-hexyl acetate, ethyl propionate, butyl propionate, isobutyl propionate, isoamyl propionate, ethyl butyrate, ethyl 2-methylbutyrate, butyl isobutyrate, ethyl hexanoate, ethyl pivalate, methyl 4-methylvalerate, ethyl valerate, ethyl isovalerate, isopropyl isovalerate, ethyl lactate, 2-pentanone, heptanoic acid, 3-heptanone, 2,3-hexanedione, d-camphor, p-methyl anisole, 2-methoxy-6-methyl pyrazine, 1,8-cineole, 1,4-cineole, benzyl methyl ether, 3-heptanol, isoamyl isovalerate, butyl valerate, butyl lactate, methyl benzoate, ethyl benzoate, propyl benzoate, butyl benzoate, isobutyl benzoate, amyl benzoate, isoamyl benzoate, methyl phenylacetate, ethyl phenylacetate, benzyl acetate, sassafras acetate, isobornyl acetate, ethyl 3-phenylpropionate, 2-methyl-5-ethoxypyrazine, 2-methyl-6-ethoxypyrazine, 2,3-diethyl-5-methylpyrazine, 5-methyl-6,7-dihydro-5H-cyclopentapyrazine, 2-acetyl pyrazine, acetyl pyridine, 2-acetyl pyridine, 3-acetyl pyridine, 2-t-butyl cyclohexanone, p-dimethyl-hydroquinone, isoquinoline, 2,3-dimethyl-benzofuran, 3,6-dimethyl benzofuranone, ambernaphthofuran, p-t-butyl-cyclohexanone, thymol methyl ether, 3-phenylpropyl alcohol, 2,6-dimethoxyphenol, 2-t-butyl-6-methyl-phenol, 1-menthol, dl-menthol, d-neomenthol, menthone lactone, p-ethyl acetophenone, skatole, diphenyl ether, β-naphthyl methyl ether, β-naphthyl ethyl ether, saffron indenone, ambroxide, (-)-ambroxide, phenylacetic acid, watermelon ketone, dihydroactinidiolide, 3-butyl-phthalide, borneol, dl-isoborneol, thymol, exaltolide, exaltone, menthyl lactate, methyl dihydrojasmonate, ethylene brassylate, t-hydrofurfuryl phenylacetate, 2-(3-phenylpropyl)pyridine), (±)-muscone, (-)-muscone, isomuscone, normuscone, cyclohexadecanone, celestolide, sandal cyclopropane, aniseed oil, eucalyptus oil, peppermint oil, and spearmint oil.

More preferably, the fragrant substance is composed of: at least one fragrant substance belonging to fragrant substance group A having a vapor pressure of 1.0-120 mmHg at normal temperature and being selected from butyl acetate, amyl acetate, isoamyl acetate, hexyl acetate, 2-hexyl acetate, ethyl propionate, butyl propionate, isobutyl propionate, isoamyl propionate, ethyl butyrate, ethyl 2-methylbutyrate, butyl isobutyrate, ethyl hexanoate, ethyl pivalate, methyl 4-methylvalerate, ethyl valerate, ethyl isovalerate, isopropyl isovalerate, ethyl lactate, 2-pentanone, heptanoic acid, 3-heptanone, 2,3-hexanedione, d-camphor, p-methyl anisole, 2-methoxy-6-methyl pyrazine, 1,8-cineole, 1,4-cineole, benzyl methyl ether, and 3-heptanol;
at least one fragrant substance belonging to fragrant substance group B having a vapor pressure of 0.001-1.0 mmHg at normal temperature and being selected from isoamyl isovalerate, butyl valerate, butyl lactate, methyl benzoate, ethyl benzoate, propyl benzoate, butyl benzoate, isobutyl benzoate, amyl benzoate, isoamyl benzoate, methyl phenylacetate, ethyl phenylacetate, benzyl acetate, sassafras acetate, isobornyl acetate, ethyl 3-phenylpropionate, 2-methyl-5-ethoxypyrazine, 2-methyl-6-ethoxypyrazine, 2,3-diethyl-5-methylpyrazine, 5-methyl-6,7-dihydro-5H-cyclopentapyrazine, 2-acetyl pyrazine, acetyl pyridine, 2-acetyl pyridine, 3-acetyl pyridine, 2-t-butyl cyclohexanone, p-dimethyl-hydroquinone, isoquinoline, 2,3-dimethyl-benzofuran, 3,6-dimethyl benzofuranone, ambernaphthofuran, p-t-butyl-cyclohexanone, thymol methyl ether, 3-phenylpropyl alcohol, 2,6-dimethoxyphenol, 2-t-butyl-6-methyl-phenol, 1-menthol, dl-menthol, d-neomenthol, menthone lactone, p-ethyl acetophenone, skatole, diphenyl ether, β-naphthyl methyl ether, β-naphthyl ethyl ether, saffron indenone, ambroxide, (-)-ambroxide, phenylacetic acid, watermelon ketone, dihydroactinidiolide, 3-butyl-phthalide, borneol, dl-isoborneol, thymol, aniseed oil, eucalyptus oil, peppermint oil, and spearmint oil; and
at least one fragrant substance belonging to fragrant substance group C having a vapor pressure of less than 0.001 mmHg at normal temperature and being selected from exaltolide, exaltone, menthyl lactate, methyl dihydrojasmonate, ethylene brassylate, t-hydrofurfuryl phenylacetate, 2-(3-phenylpropyl)pyridine, (±)-muscone, (-)-muscone, isomuscone, normuscone, cyclohexadecanone, celestolide, and sandal cyclopropane.

Most preferably, the fragrant substance belonging to fragrant substance group A having a vapor pressure of 1.0-120 mmHg at normal temperature is at least one selected from the group consisting of amyl acetate, isoamyl acetate, 2-hexyl acetate, ethyl propionate, butyl propionate, isobutyl propionate, ethyl butyrate, ethyl 2-methylbutyrate, butyl isobutyrate, methyl 4-methylvalerate, ethyl valerate, ethyl isovalerate, isopropyl isovalerate, 2-pentanone, 2,3-hexanedione, d-camphor, 1,8-cineole, and 1,4-cineole; the fragrant substance belonging to fragrant substance group B having a vapor pressure of 0.001-1.0 mmHg at normal temperature is at least one selected from the group consisting of butyl valerate, ethyl benzoate, propyl benzoate, isobornyl acetate, isoquinoline, ambernaphthofuran, l-menthol, d-neomenthol, diphenyl ether, β-naphthyl ethyl ether, ambroxide, and (-)-ambroxide; and the fragrant substance belonging to fragrant substance group C having a vapor pressure of less than 0.001 mmHg at normal temperature is selected from the group consisting of exaltolide, (±)-muscone, and (-)-muscone.

In still another aspect, the composition of the present invention prevents or treats several respiratory infectious diseases by inactivating viruses or bacteria causing respiratory infectious diseases and the respiratory infectious disease are preferably viral infectious diseases. Especially, the composition of the present invention can promptly alleviate or remove systemic or local symptoms occurring due to an infection, such as a cold or influenza, that is, symptoms, such as sneezing, runny nose, stuffy nose, breathing problems, itchy eyes or tearing eyes, facial pressure, coughing, and inflammation; pains, such as sore throat, muscular pain, and joint pain; and symptoms, such as fever, chills, headache, discomfort, fatigue, lethargy, anorexia, drowsiness, and malaise.

Representative viruses causing an infection in the respiratory tract may include adenovirus, influenza virus, parainfluenza virus, respiratory syncytial virus, rhinovirus, and coronavirus.

The influenza viruses are divided into types A, B, and C viruses. Compared with the type B or C viruses, the type A viruses are confirmed to infect mainly in humans, and confirmed to infect pigs, other mammals, and various wild birds. Avian influenza virus, swine influenza virus, and influenza A virus subtype H1N1, which have recently become a problem worldwide, belong to the type A viruses.

The coronaviruses were confirmed to infect mammals, such as dogs, pigs, and cows, and birds, and may include MERS-coronavirus, SARS-coronavirus, coronavirus 229E, coronavirus OC43, coronavirus NL63, canine coronavirus, bovine coronavirus, porcine respiratory coronavirus, porcine epidemic diarrhea virus, avian infectious bronchitis virus, and the like.

Besides, the viruses causing an infection in the respiratory tract include enterovirus, metapneumovirus, bocavirus, coxsackievirus, and the like, but are not particularly limited thereto.

Representative viruses causing an infection in the respiratory tract include *Streptococcus pneumoniae, Haemophilus influenza, and further include Staphylococcus aureus, Moraxella catarrhalis, pneumococcus, Pseudomonas aeruginosa, Serratia marcescens, Burkholderia cepacia, Escherichia coli, Mycobacterium avium, Klebsiella pneumoniae, Chlamydia pneumoniae, Legionella pneumophila, Porphyromonas gingivalis,* and *Acinetobacter baumannii.*

The composition of the present invention causes no pain and irritation in the respiratory tract mucosa tissue, and thus can be applied to all tissues and connective organs of the respiratory tract, that is, at least one of eyes, lacrimal ducts, oral cavity, nasal cavity, paranasal sinuses, nasopharynx, oropharynx, laryngopharynx, tonsils, trachea, bronchus, bronchioles, and lungs, and preferably all the sites of eyes, lacrimal ducts, oral cavity, nasal cavity, paranasal sinuses, nasopharynx, oropharynx, laryngopharynx, tonsils, trachea, bronchus, bronchioles, and lungs.

The composition of the present invention, when applied to the above sites, may be used in the form of a liquid or a spray. When the site of application is the eyes, oral cavity, nasal cavity, and paranasal sinuses, the composition is preferably applied in the form of a liquid. When the site of application is the nasal cavity, the composition is preferably applied to the nasal cavity in the form of a liquid and then the nasal cavity is subjected to vibration, pulse, pressure variation, or liquid shaking. In addition, the composition is preferably applied in the form of a spray when the site of application is the nasopharynx, oropharynx, laryngopharynx, tonsils, trachea, bronchus, bronchioles, and lungs.

In addition, the composition of the present invention can be administered using a tool (e. g., a spray, a syringe, a squeeze bottle, etc.) suitable for the application of the composition in the form of a liquid or a spray, but is not particularly limited thereto.

However, more preferably, as for the administration of the composition of the present invention into eyes, one drop of the composition is placed in each eye by an eye dropper, and then the eyelids are rubbed several times with cleanly washed fingers. Additionally, one drop of the composition is placed in each eye, and then blinking is performed ten times, and then the solution is sucked into the nose for about 15 seconds while the nose is held with a hand. Such a procedure is repeated two times.

As for the administration to the nasal cavity, the nose is blown in front of a sink, a needleless syringe is filled with 10 ml of an aqueous solution of an example, the head is leaned forward horizontally, the face is turned horizontally, the syringe is kept close to a nostril so as to prevent the solution from leaking into the lower portion of the nostril, and then the solution is slowly injected into the nasal cavity. If the solution does not enter the nostril well due to nasal congestion, the solution is slowly pushed while the thumb press of the syringe is strongly pressed. After the solution is fully injected, the syringe is kept close to the nostril so as to prevent the solution from leaking while the position is held, and then in the same position, the pressing and releasing of the nose ball through the syringe is rapidly repeated for 30 seconds, so that the solution in the nose is kept running. The repetitive pressing and releasing of the nose ball is consecutively performed for 3 minutes or longer if nasal congestion is serious. The same is also performed on the opposite nostril. The above procedure may be repeated once more where nasal congestion remains.

As for the administration to the nasopharynx, a syringe or spray with a nasopharyngeal injection nozzle, containing 10-20 ml of the composition is held such that the spray direction is the upward direction, with the head tilted forward about 45 degrees, and the nozzle is inserted deep so as to touch the nasopharynx wall, and then the composition is sprayed behind the throat and to the nasopharynx. The tip of the nozzle is slightly moved so as to apply the spray throughout the nasopharynx, so that the spray is applied to the entire surfaces of the back of the soft palate, the nasopharynx, and the retronasal route.

As for the administration to the tonsils, 10 ml of the composition of the present invention is placed in a syringe or spray with a nasopharyngeal injection nozzle and then 10 ml of the composition is concentrated little by little on the painful inflammation site over one minute or longer.

As for the administration to the trachea, bronchus, bronchioles, and lungs, a nozzle of a spray pump containing 10 ml of the composition is placed into the mouth in the upright position, and then lips are narrowed extremely and the inside of the neck is widened as much as possible. Thereafter, the composition is sprayed toward the throat while a strong breath is taken through the mouth. The spray enters the trachea, bronchus, bronchioles, and alveoli along the inhaled air.

As for the administration to the oral cavity, the composition is placed in the mouth, and gargling is performed for about 30 seconds while the head is raised so that the composition touches the tonsils and larynx.

In order to increase the treatment effect on respiratory infectious diseases, it is most preferable that within 1 hour after the completion of the application of the above methods to the eyes, oral cavity, nasal cavity, paranasal sinuses, tonsils, trachea, bronchus, bronchioles, and lungs, the application is repeated once or twice.

The application of the above methods to patients with respiratory infectious diseases, especially, a cold or flu, can promptly relieve or eliminate symptoms, such as headache, runny nose, nose congestion, and fever, within 1-4 hours after administration, depending on the severity of infection, so that daily activities of the patients are not disturbed and recurrence or deterioration of the diseases is prevented until several days later.

The dose may vary depending on the age, sex, or body weight of a subject to be treated, the particular disease or pathological condition to be treated, the severity of the disease or pathological condition, the time of administration, the route of administration, the absorption, distribution, and excretion rate of a drug, the kind of another drug used, and the determination of a prescriber. The determination of dose on the basis of the factors is within the level of a person skilled in the art, and the administration may be performed once daily or divided several times. The dose is not intended to limit the scope of the invention in any aspect.

### Advantageous Effects

The present invention is directed to a solid composition containing an iodine agent and sodium chloride and having improved water solubility and to an antiviral and antibacterial ocular, oral, nasal, or inhalation composition containing an aqueous solution of the solid composition, wherein the solid composition contains a mixture of an iodine agent and sodium chloride or an iodine, sodium chloride, and a fragrant substance and has excellent stability as well as water solubility, such as a dissolution rate of the solid composition in water being 30 seconds or less.

Furthermore, the aqueous solution obtained by dissolving the solid composition in water can be helpfully used in the prevention or treatment of respiratory infectious diseases since the aqueous solution has excellent effects of masking an unpleasant or repulsive smell and taste of the iodine agent, does not cause pain or irritation, has excellent effects of preventing or treating a cold or influenza as a respiratory infectious disease, and can be applied to all the organ sites of the respiratory tract.

### Mode for Carrying Out the Invention

Hereinafter, preferable embodiments of the present invention will be described in detail. However, the present invention is not limited to the embodiments described herein, and can be embodied in many different forms. Rather, these embodiments are provided so that this disclosure will be thorough and complete and will fully convey the scope of the disclosure to those skilled in the art.

### <Example 1: Preparation of povidone iodine and sodium chloride-mixed solid composition with improved stability and dissolubility>

Referring to Table 1 below, povidone iodine (poly(vinylpyrrolidone)-Iodine complex, 10:1, Sigma-Aldrich), and sodium chloride pulverized to 30 mesh or more (600 µm or less), and a fragrant substance were placed in a mortar, followed by homogeneous pulverization for 5 minutes, thereby preparing a powder-form solid composition with improved stability and solubility.

**[Table 1]**

| Condition | Povidone iodine (g) | Sodium chloride (g) | Fragrant substance (g) | |
|---|---|---|---|---|
| | | | Used fragrant substance (g) | Total amount of fragrant substance used (g) |
| Example 1-1 | 0.2 | 1.5 | - | - |
| Example 1-2 | 0.2 | 2.5 | - | - |
| Example 1-3 | 0.2 | 3.5 | - | - |
| Example 1-4 | 0.1 | 1.5 | - | - |
| Example 1-5 | 0.1 | 3.5 | - | - |
| Example 1-6 | 0.3 | 1.5 | - | - |
| Example 1-7 | 0.3 | 3.5 | - | - |
| Example 1-8 | 0.01 | 2.5 | - | - |
| Example 1-9 | 0.5 | 2.0 | - | - |
| Example 1-10 | 0.5 | 1.5 | - | - |
| Example 1-11 | 0.1 | 2.5 | isoamyl acetate 0.001, l-memthol 0.002, (-)-ambroxide 0.0006, exaltolide 0.0003 | 0.0039 |
| Example 1-12 | 0.1 | 2.5 | d-camphor 0.001, 1,8-cineole 0.005, l-memthol 0.002, (-)-ambroxide 0.0004, exaltolide 0.0002 | 0.0086 |
| Example 1-13 | 0.2 | 2.5 | isoamyl acetate 0.001, l-memthol 0.002, (-)-ambroxide 0.0006, exaltolide 0.0003 | 0.0039 |
| Example 1-14 | 0.2 | 2.5 | d-camphor 0.001, 1,8-cineole 0.005, l-memthol 0.002, (-)-ambroxide 0.0006, exaltolide 0.0003 | 0.0089 |
| Example 1-15 | 0.2 | 2.5 | 2-hexyl acetate 0.0005, butyl propionate 0.0005, butyl isobutyrate 0.0005, ethyl valerate 0.0005, 2-pentanone 0.0005, 1-memthol 0.001, (-)-ambroxide 0.0004, exaltolide 0.0002 | 0.0041 |
| Example 1-16 | 0.2 | 2.5 | isoamyl acetate 0.001, butyl valerate 0.0005, isobornyl acetate 0.0005, isoquinoline 0.0003, ambernaphthofuran 0.0003, exaltolide 0.0002 | 0.0028 |
| Example 1-17 | 0.3 | 2.5 | isoamyl acetate 0.001, l-memthol 0.002, (-)-ambroxide 0.0006, exaltolide 0.0003 | 0.0039 |

### <Example 2: Preparation of antiviral and antibacterial ocular, oral, nasal, or inhalation composition containing povidone iodine and sodium chloride>

Referring to Table 2 below, povidone iodine (poly(vinylpyrrolidone)-Iodine complex, 10:1, Sigma-Aldrich), sodium chloride, and a fragrant substance were placed in a mortar, followed by homogeneous pulverization, and then distilled water was added to reach 100 ml in the total amount of the composition, followed by dissolution.

The antiviral and antibacterial ocular, oral, nasal, or inhalation compositions of Examples 2-1 to 2-44 were obtained.

**[Table 2]**

| Condition | Povidone iodine (g) | Sodium chloride (g) | Fragrant substance | | | Total amount (mℓ) |
|---|---|---|---|---|---|---|
| | | | Fragrant substance group A^{a}(g) | Fragrant substance group B^{b}(g) | Fragrant substance group C^{c}(g) | |
| Example 2-1 | 0.2 | 1.2 | - | - | - | 100 |
| Example 2-2 | 0.2 | 2.6 | - | - | - | 100 |
| Example 2-3 | 0.2 | 3.5 | - | - | - | 100 |
| Example 2-4 | 0.1 | 1.2 | - | - | - | 100 |
| Example 2-5 | 0.1 | 3.5 | - | - | - | 100 |
| Example 2-6 | 0.25 | 1.2 | - | - | - | 100 |
| Example 2-7 | 0.25 | 3.5 | - | - | - | 100 |
| Example 2-8 | 0.1 | 2.6 | isoamyl acetate 0.003 | - | - | 100 |
| Example 2-9 | 0.1 | 2.6 | - | l-memthol 0.002, diphenyl ether 0.0002 | - | 100 |
| Example 2-10 | 0.1 | 2.6 | - | l-memthol 0.002, β-naphthylethyl ether 0.0002 | - | 100 |
| Example 2-11 | 0.1 | 2.6 | - | l-memthol 0.0018, (-)-ambroxide 0.0004 | - | 100 |
| Example 2-12 | 0.1 | 2.6 | - | - | exaltolide 0.0002 | 100 |
| Example 2-13 | 0.1 | 2.6 | 1,8-cineole 0.002 | l-memthol 0.002 | - | 100 |
| Example 2-14 | 0.1 | 2.6 | ethyl butyrate 0.0003 | (-)-ambroxide 0.0002 | - | 100 |
| Example 2-15 | 0.1 | 2.6 | - | l-memthol 0.002 | exaltolide 0.0001 | 100 |
| Example 2-16 | 0.1 | 2.6 | - | l-memthol 0.0015, (-)-ambroxide 0.0004 | exaltolide 0.0002 | 100 |
| Example 2-17 | 0.1 | 2.6 | isoamyl acetate 0.001 | l-memthol 0.002, (-)-ambroxide 0.0006 | exaltolide 0.0003 | 100 |
| Example 2-18 | 0.1 | 2.6 | d-camphor 0.001, 1,8-cineole 0.005 | l-memthol 0.002, (-)-ambroxide 0.0004 | exaltolide 0.0002 | 100 |
| Example 2-19 | 0.1 | 2.6 | ethyl propionate 0.001 | l-memthol 0.001, (-)-ambroxide 0.0004 | exaltolide 0.0002 | 100 |
| Example 2-20 | 0.1 | 1.2 | isoamyl acetate 0.001 | l-memthol 0.002, (-)-ambroxide 0.0006 | exaltolide 0.0003 | 100 |
| Example 2-21 | 0.1 | 3.0 | isoamyl acetate 0.001 | l-memthol 0.002, (-)-ambroxide 0.0006 | exaltolide 0.0003 | 100 |
| Example 2-22 | 0.2 | 2.6 | isoamyl acetate 0.003 | - | - | 100 |
| Example 2-23 | 0.2 | 2.6 | ethyl butyrate 0.0003 | - | - | 100 |
| Example 2-24 | 0.2 | 2.6 | d-camphor 0.002, 1,8-cineole 0.006 | - | - | 100 |
| Example 2-25 | 0.2 | 2.6 | - | propyl benzoate 0.0005 | - | 100 |
| Example 2-26 | 0.2 | 2.6 | - | l-memthol 0.002, (-)-ambroxide 0.0004 | - | 100 |
| Example 2-27 | 0.2 | 3.0 | - | l-memthol 0.002, (-)-ambroxide 0.0004 | - | 100 |
| Example 2-28 | 0.2 | 2.6 | - | - | exaltolide 0.0002 | 100 |
| Example 2-29 | 0.2 | 2.6 | isoamyl acetate 0.003 | l-memthol 0.002, (-)-ambroxide 0.0006 | - | 100 |
| Example 2-30 | 0.2 | 2.6 | - | l-memthol 0.002, (-)-ambroxide 0.0004 | exaltolide 0.0002 | 100 |
| Example 2-31 | 0.2 | 2.6 | isoamyl acetate 0.001 | l-memthol 0.002, (-)-ambroxide 0.0006 | exaltolide 0.0003 | 100 |
| Example 2-32 | 0.2 | 2.6 | d-camphor 0.001, 1,8-cineole 0.005 | l-memthol 0.002, (-)-ambroxide 0.0006 | exaltolide 0.0003 | 100 |
| Example 2-33 | 0.2 | 2.6 | ethyl propionate 0.003 | l-memthol 0.002, (-)-ambroxide 0.0006 | exaltolide 0.0003 | 100 |
| Example 2-34 | 0.2 | 2.6 | isoamyl acetate 0.003, ethyl butyrate 0.003 | l-memthol 0.001, (-)-ambroxide 0.0004 | exaltolide 0.0002 | 100 |
| Example 2-35 | 0.2 | 2.6 | 2-hexyl acetate 0.0005, butyl propionate 0.0005, butyl isobutyrate 0.0005, ethyl valerate 0.0005, 2-pentanone 0.0005 | l-memthol 0.001, (-)-ambroxide 0.0004 | exaltolide 0.0002 | 100 |
| Example 2-36 | 0.2 | 2.6 | isoamyl acetate 0.001 | butyl valerate 0.0005, isobornyl acetate 0.0005, isoquinoline 0.0003, ambernaphthofuran 0.0003 | exaltolide 0.0002 | 100 |
| Example 2-37 | 0.2 | 2.6 | isoamyl acetate 0.001 | l-memthol 0.002, (-)-ambroxide 0.0006 | (±)-muscone 0.0003 | 100 |
| Example 2-38 | 0.2 | 1.2 | isoamyl acetate 0.001 | l-memthol 0.002, (-)-ambroxide 0.0006 | exaltolide 0.0003 | 100 |
| Example 2-39 | 0.2 | 3.0 | isoamyl acetate 0.001 | l-memthol 0.002, (-)-ambroxide 0.0006 | exaltolide 0.0003 | 100 |
| Example 2-40 | 0.25 | 2.6 | isoamyl acetate 0.003 | - | - | 100 |
| Example 2-41 | 0.25 | 2.6 | - | l-memthol 0.002, (-)-ambroxide 0.0006 | exaltolide 0.0003 | 100 |
| Example 2-42 | 0.25 | 2.6 | isoamyl acetate 0.001 | l-memthol 0.002, (-)-ambroxide 0.0006 | exaltolide 0.0003 | 100 |
| Example 2-43 | 0.25 | 1.2 | isoamyl acetate 0.001 | l-memthol 0.002, (-)-ambroxide 0.0006 | exaltolide 0.0003 | 100 |
| Example 2-44 | 0.25 | 3.0 | isoamyl acetate 0.001 | l-memthol 0.002, (-)-ambroxide 0.0006 | exaltolide 0.0003 | 100 |
| ^{a} Fragrant substance group A: fragrant substances having a vapor pressure of 1.0-120 mmHg at normal temperature | | | | | | |
| ^{b} Fragrant substance group B: fragrant substances having a vapor pressure of 0.001-1.0 mmHg at normal temperature | | | | | | |
| ^{c} Fragrant substance group C fragrant substances having a vapor pressure of less than 0.001 mmHg at normal temperature | | | | | | |

### <Comparative Example 1: Preparation of comparative povidone iodine and sodium chloride-mixed solid composition>

A solid composition of Comparative Example 1 was prepared in the same manner as in Example 1, with reference to Table 3 below.

**[Table 3]**

| Condition | Povidone iodine (g) | Sodium chloride (g) | Fragrant substance (g) | |
|---|---|---|---|---|
| | | | Used fragrant substance (g) | Total amount of fragrant substance used (g) |
| Comparative Example 1-1 | 0.2 | - | - | - |
| Comparative Example 1-2 | 2.5 | 2.5 | - | - |
| Comparative Example 1-3 | 5 | 2.5 | - | - |
| Comparative Example 1-4 | 0.2 | 0.2 | - | - |
| Comparative Example 1-5 | 0.2 | 0.1 | - | - |
| Comparative Example 1-6 | 0.2 | - | isoamyl acetate 0.001, l-memthol 0.002, (-)-ambroxide 0.0006, exaltolide 0.0003 | 0.0039 |
| Comparative Example 1-7 | 0.2 | sodium sulfate anhydride 2.5 | - | - |
| Comparative Example 1-8 | 0.2 | ammonium chloride 2.5 | - | - |
| Comparative Example 1-9 | 0.2 | potassium chloride 2.5 | - | - |
| Comparative Example 1-10 | 0.2 | potassium bromide 2.5 | - | - |
| Comparative Example 1-11 | 0.2 | D-mannitol 2.5 | - | - |

### <Comparative Example 2: Preparation of comparative antiviral and antibacterial ocular, oral, nasal, or inhalation composition containing povidone iodine and sodium chloride>

A comparative ocular, oral, nasal, or inhalation antiviral and antibacterial composition of Comparative Example 2 was prepared in the same manner as in Example 2, with reference to Table 4 below.

**[Table 4]**

| Condition | [Povidone iodine (g) | Sodium chloride (g) | Fragrant substance | | | Total amount (mℓ) |
|---|---|---|---|---|---|---|
| | | | Fragrant substance group A^{a} (g) | Fragrant substance group B^{b} (g) | Fragrant substance group C^{c} (g) | Total amount (mℓ) |
| Comparative Example 2-1 | 0.2 | - | - | - | - | 100 |
| Comparative Example 2-2 | 0.001 | 2.5 | - | - | - | 100 |
| Comparative Example 2-3 | 2.0 | 2.5 | - | - | - | 100 |
| Comparative Example 2-4 | 0.2 | 0.05 | - | - | - | 100 |
| Comparative Example 2-5 | 0.2 | 10.0 | - | - | - | 100 |
| Comparative Example 2-6 | 0.2 | - | isoamyl acetate 0.001 | 1-memthol 0.002, (-)-ambroxide 0.0006 | exaltolide 0.0003 | 100 |
| Comparative Example 2-7 | 0.001 | 2.6 | isoamyl acetate 0.001 | l-memthol 0.002, (-)-ambroxide 0.0006 | exaltolide 0.0003 | 100 |
| Comparative Example 2-8 | 2.0 | 2.6 | isoamyl acetate 0.001 | l-memthol 0.002, (-)-ambroxide 0.0006 | exaltolide 0.0003 | 100 |
| Comparative Example 2-9 | 0.2 | 0.05 | isoamyl acetate 0.001 | l-memthol 0.002, (-)-ambroxide 0.0006 | exaltolide 0.0003 | 100 |
| Comparative Example 2-10 | 0.2 | 10.0 | isoamyl acetate 0.001 | l-memthol 0.002, (-)-ambroxide 0.0006 | exaltolide 0.0003 | 100 |
| Comparative Example 2-11 | 0.2 | 2.6 | isoamyl acetate 0.000005 | l-memthol 0.00002, (-)-ambroxide 0.000006 | exaltolide 0.000003 | 100 |
| Comparative Example 2-12 | 0.2 | 2.6 | isoamyl acetate 0.05 | l-memthol 0.2, (-)-ambroxide 0.06 | exaltolide 0.03 | 100 |
| ^{a} Fragrant substance group A: fragrant substances having a vapor pressure of 1.0-120 mmHg at normal temperature | | | | | | |
| ^{b} Fragrant substance group B: fragrant substances having a vapor pressure of 0.001-1.0 mmHg at normal temperature | | | | | | |
| ^{c} Fragrant substance group C fragrant substances having a vapor pressure of less than 0.001 mmHg at normal temperature | | | | | | |

### <Test Example 1: Investigation of storage stability>

To investigate the storage stability of the solid compositions of the present invention, Examples 1-2 and 1-11 were placed in a mortar, followed by pulverization for 3 minutes, thereby preparing a homogeneous mixture. The mixture was placed in a 5-ml brown glass vial, which was then covered with a lid, and then stored in an incubator at 60°C for 2 weeks. The loss of iodine was determined at weekly intervals. Examples 1-2 and 1-11 were repeatedly tested three times, and the temperature condition of 60°C is a strict condition for relative comparison of drug stability.

To determine the amount of iodine lost, each sample was taken out at weekly intervals, and the mixture contained in the 5-ml glass vial was dissolved in 100 ml of distilled water, and titrated with a 0.01 N sodium thiosulfate standard solution (product from Samchun Pure Chemical) for quantification. The amount of iodine lost is calculated by 1.269 mg of iodine per ml of the 0.01 N sodium thiosulfate standard solution, and the calculation results are shown in Table 5.

**[Table 5]**

| Condition | Amount of iodine lost (%) | | |
|---|---|---|---|
| Condition | Early | After 1 week | After 2 weeks |
| Example 1-2 | 0 | 0.67 | 1.04 |
| Example 1-11 | 0 | 0.75 | 1.30 |
| Aqeous solution in which Example 1-2 is dissolved in 100 ml of water | 0 | 33.0 | 65.5 |

Referring to Table 5 above, the solid compositions of Examples 1-2 and 1-11 showed very small amounts of iodine lost, within 1.5%, for 2 weeks even in the high-temperature condition of 60°C. However, as a result of measuring the amount of iodine lost for 2 weeks in a high-concentration temperature of 60°C from an aqueous solution obtained by dissolving the solid composition of Example 1-2 in water, the amount of iodine lost was 50 times or more compared with the amount of iodine lost from the solid composition, and the final concentration of povidone iodine remaining was 0.1% or less, indicating that the aqueous solution had very low storage stability.

It can be therefore verified that a solid composition containing a mixture of a low-concentration iodine agent and sodium chloride like in the present invention remains with an effective amount even for a long-term storage, indicating that the solid composition does not lose its activity and has high storage stability.

### <Test Example 2: Investigation of dissolution rate in water>

Each of the compositions of Comparative Example 1 and Comparative Example 1 was placed in a 100-ml glass vial, and 100 ml of distilled water of 25°C (± 0.5°C) was added, followed by stirring at 300 rpm in a magnetic stirrer. The examples and comparative examples were repeatedly tested three times. To determine the time taken for povidone iodine to completely dissolve in water, the time from when distilled water was added to form a solution to when the residue of povidone iodine is completely removed from the container wall was measured. The measurements are shown in Table 6 below.

**[Table 6]**

| Condition | Completely dissolved time (sec) |
|---|---|
| Example 1-1 | 12 |
| Example 1-2 | 10 |
| Example 1-3 | 7 |
| Example 1-4 | 8 |
| Example 1-5 | ≤5 |
| Example 1-6 | 13 |
| Example 1-7 | 8 |
| Example 1-8 | ≤5 |
| Example 1-9 | 15 |
| Example 1-10 | 23 |
| Example 1-11 | ≤5 |
| Example 1-12 | ≤5 |
| Example 1-13 | 10 |
| Example 1-14 | 10 |
| Example 1-15 | 10 |
| Example 1-16 | 10 |
| Example 1-17 | 12 |
| Comparative Example 1-1 | 900 |
| Comparative Example 1-2 | 640 |
| Comparative Example 1-3 | 650 |
| Comparative Example 1-4 | 600 |
| Comparative Example 1-5 | 630 |
| Comparative Example 1-6 | 930 |
| Comparative Example 1-7 | 1044 |
| Comparative Example 1-8 | 60 |
| Comparative Example 1-9 | 60 |
| Comparative Example 1-10 | 120 |
| Comparative Example 1-11 | 80 |

Referring to Table 6 above, the solid compositions of the examples of the present invention had a dissolution rate of 30 seconds or less in water, indicating that the solid compositions dissolve in water very fast.

However, in the absence of sodium chloride as in Comparative Examples 1-1 and 1-6, the dissolution rate in water was 900 seconds or longer, that is, the dissolution takes 15 minutes or longer. Comparative Example 1-7 using sodium sulfate instead of sodium chloride showed a very low dissolution rate in water, taking at least 1044 seconds (about 17 minutes). Comparative Example 1-8 using ammonium chloride, Comparative Example 1-9 using potassium chloride, Comparative Example 1-10 using potassium bromide, and Example 1-11 using D-mannitol took 60-120 seconds, showing significantly low dissolution rates in water compared with the examples of the present invention.

Although not shown in Table 6 above, the solid composition, when containing less than 0.2 parts by weight of povidone iodine relative to 100 parts by weight of sodium chloride, was not suitable for use since such a solid composition had a fast dissolution rate in water but the povidone iodine was contained below the effective content to exhibit antiviral and antibacterial effects.

It can be therefore seen that a solid composition containing a mixture of an iodine agent and sodium chloride like in the present invention does not lose its activity even for a long-term storage, has high storage stability, is not affected by the place, such as home or hospital, and has an excellent dissolution rate in water, and thus the solid composition is not at all inconvenient to use.

### <Test Example 3: Sensory evaluation>

To make a sensory evaluation on the antiviral and antibacterial ocular, oral, nasal, or inhalation compositions containing an iodine agent, sodium chloride, and a fragrant substance, manufactured in Example 2 and Comparative Example 2 of the present invention, 20 trained panels rated each of the compositions as very good (5 points), good (4 points), moderate (3 points), dislike (2 points), very dislike (1 point) by using the 5-point scale method. Table 7 shows each average of scores rated by the 20 panels to one decimal place.

The degree of satisfaction in the effect of masking an unpleasant or repulsive smell the iodine agent when each of the compositions of the examples and comparative examples was placed in a spray container and sprayed in the oral cavity once to three times and the effect of masking an unpleasant or repulsive taste of the iodine agent when 30 ml of each of the compositions of the examples and comparative examples was placed in the oral cavity and gargled for 30 seconds were measured. The higher the masking effect against an unpleasant or repulsive smell or taste, the higher the score was evaluated (5 points). In addition, when 10 ml of each of the compositions of examples and comparative examples was injected into the nasal cavity through a syringe, the greater the nasal irritation was, the lower the score was evaluated (1 point). The overall preference was entirely measured.

**[Table 7]**

| Condition | Satisfaction on composition containing iodine agent, sodium chloride, and fragrant substance | | | Overall preference |
|---|---|---|---|---|
| | Smell masking effect | Taste masking effect | Nasal irritation | |
| Example 2-1 | 2.1 | 2.9 | 4.7 | 3.2 |
| Example 2-2 | 2.3 | 3.1 | 4.8 | 3.4 |
| Example 2-3 | 2.7 | 3.2 | 4.9 | 3.6 |
| Example 2-4 | 2.6 | 3.4 | 4.8 | 3.6 |
| Example 2-5 | 2.7 | 3.5 | 4.9 | 3.7 |
| Example 2-6 | 1.8 | 2.7 | 4.5 | 3.0 |
| Example 2-7 | 2.2 | 3.2 | 4.4 | 3.3 |
| Example 2-8 | 3.5 | 3.7 | 4.8 | 4.0 |
| Example 2-9 | 3.9 | 4.0 | 4.7 | 4.2 |
| Example 2-10 | 3.8 | 4.0 | 4.8 | 4.2 |
| Example 2-11 | 4.0 | 4.0 | 5.0 | 4.3 |
| Example 2-12 | 3.7 | 3.5 | 4.9 | 4.0 |
| Example 2-13 | 4.1 | 4.0 | 5.0 | 4.4 |
| Example 2-14 | 3.9 | 4.2 | 5.0 | 4.4 |
| Example 2-15 | 4.1 | 4.1 | 5.0 | 4.4 |
| Example 2-16 | 3.8 | 4.2 | 5.0 | 4.3 |
| Example 2-17 | 5.0 | 5.0 | 5.0 | 5.0 |
| Example 2-18 | 4.8 | 4.8 | 5.0 | 4.9 |
| Example 2-19 | 4.7 | 4.7 | 5.0 | 4.8 |
| Example 2-20 | 4.8 | 4.9 | 5.0 | 4.9 |
| Example 2-21 | 4.9 | 5.0 | 5.0 | 5.0 |
| Example 2-22 | 4.0 | 3.9 | 5.0 | 4.3 |
| Example 2-23 | 3.4 | 3.8 | 5.0 | 4.1 |
| Example 2-24 | 3.8 | 4.1 | 4.9 | 4.3 |
| Example 2-25 | 4.1 | 3.8 | 5.0 | 4.3 |
| Example 2-26 | 4.2 | 4.1 | 5.0 | 4.4 |
| Example 2-27 | 4.1 | 4.0 | 4.9 | 4.3 |
| Example 2-28 | 3.3 | 3.6 | 5.0 | 4.0 |
| Example 2-29 | 4.2 | 4.1 | 5.0 | 4.4 |
| Example 2-30 | 4.2 | 4.2 | 5.0 | 4.5 |
| Example 2-31 | 5.0 | 4.9 | 5.0 | 5.0 |
| Example 2-32 | 4.9 | 4.9 | 4.9 | 4.9 |
| Example 2-33 | 4.9 | 4.9 | 5.0 | 4.9 |
| Example 2-34 | 5.0 | 4.9 | 5.0 | 5.0 |
| Example 2-35 | 5.0 | 4.8 | 5.0 | 4.9 |
| Example 2-36 | 5.0 | 4.9 | 5.0 | 5.0 |
| Example 2-37 | 5.0 | 5.0 | 5.0 | 5.0 |
| Example 2-38 | 4.7 | 4.6 | 4.9 | 4.7 |
| Example 2-39 | 5.0 | 4.6 | 5.0 | 4.9 |
| Example 2-40 | 3.7 | 3.6 | 4.1 | 3.8 |
| Example 2-41 | 3.6 | 4.5 | 4.3 | 4.1 |
| Example 2-42 | 4.7 | 4.7 | 4.4 | 4.6 |
| Example 2-43 | 4.5 | 4.5 | 4.5 | 4.5 |
| Example 2-44 | 4.6 | 4.9 | 4.6 | 4.7 |
| Comparative Example 2-1 | 0.5 | 0.5 | 1.5 | 0.8 |
| Comparative Example 2-2 | 4.8 | 4.7 | 5.0 | 4.8 |
| Comparative Example 2-3 | 0.5 | 1.6 | 0.2 | 0.8 |
| Comparative Example 2-4 | 0.5 | 0.5 | 1.5 | 0.8 |
| Comparative Example 2-5 | 1.9 | 2.2 | 3.1 | 2.4 |
| Comparative Example 2-6 | 2.5 | 2.8 | 0.5 | 1.9 |
| Comparative Example 2-7 | 5.0 | 5.0 | 4.8 | 4.9 |
| Comparative Example 2-8 | 2.1 | 1.4 | 0.5 | 1.3 |
| Comparative Example 2-9 | 2.5 | 2.3 | 0.5 | 1.8 |
| Comparative Example 2-10 | 2.5 | 2.5 | 2.9 | 2.6 |
| Comparative Example 2-11 | 2.7 | 3.4 | 4.8 | 3.6 |
| Comparative Example 2-12 | 4.5 | 3.9 | 0.5 | 3.0 |

Referring to Table 7 above, the compositions containing an iodine agent, sodium chloride, and a fragrant substance of Examples 2-1 to 2-44 had an excellent effect of masking an unpleasant or repulsive smell and taste of the iodine agent and did not cause pain or irritation, compared with Comparative Examples 2-1 to 2-12 manufactured under different conditions from the compositions of the present invention, and thus the compositions of the present invention can be helpfully used as an antiviral and antibacterial ocular, oral, nasal, or inhalation composition.

Especially, compared with Examples 2-1 to 2-7, Examples 2-8 to 2-44 containing a fragrant substance showed a better effect in masking an unpleasant or repulsive smell and taste of the iodine agent, and the compositions containing all of fragrant substance groups A, B, and C like Examples 2-17 to 2-21, 2-31 to 2-39, and 2-42 to 2-44 showed the best masking effect.

Comparative Examples 2-2 to 2-7 are the compositions containing a low content of povidone iodine and showed an iodine smell and taste masking effect and low irritation similar to those shown by the examples of the present invention, but such compositions are not suitable for use since the compositions had a very low content of iodine and thus did not show an antiviral and antibacterial effect. On the other hand, Comparative Examples 2-3 and 2-8 are the compositions containing a high content of povidone iodine, and such compositions are not suitable for use since the smell and taste of iodine and the irritation were very strong when the compositions were administered.

It can be therefore confirmed that a composition containing an iodine agent, sodium chloride, and a fragrant substance like in the present invention has excellent antiviral and antibacterial effects, excellent effects of masking an unpleasant or repulsive smell and taste of an iodine agent, and no irritation.

### <Test Example 4: Verification on treatment effect on respiratory infectious diseases>

To investigate the treatment effect on respiratory infectious diseases by the composition manufactured according to Example 2-31 of the present invention using PVP-Iodine 30/06 (BASF), 30 cold or flu patients were administered with the composition via eyes, oral cavity, nasal cavity, paranasal sinuses, nasopharynx, oropharynx, laryngopharynx, tonsils, trachea, bronchus, bronchioles, and lungs by the application methods described below, and then the degree of recovery was checked.

Application for cold or flu treatment:
One drop of the composition was placed in each eye by an eye dropper, and then the eyelids were rubbed several times with cleanly washed fingers for 10 seconds or longer. One drop of the composition was placed in each eye, and then blinking was performed ten times, and then the solution was sucked into the nose for about 10-15 seconds while the nose was held with a hand. Such a procedure was repeated two times.

Next, in order to administer the composition into the nasal cavity, the nose was blown in front of a sink, a needleless syringe was filled with 10 ml of an aqueous solution of the example, the head was leaned forward horizontally, the face was turned horizontally, the syringe was kept close to a nostril so as to prevent the solution from leaking into the lower portion of the nostril, and then the solution was slowly injected into the nasal cavity. If the solution does not enter the nostril well due to nasal congestion, the solution is slowly pushed while the thumb press of the syringe is strongly pressed. After the solution is fully injected, the syringe is kept close to the nostril so as to prevent the solution from leaking while the position is held, and then in the same position, the pressing and releasing of the nose ball through the syringe is rapidly repeated for 30 seconds, so that the solution in the nose is kept running. The repetitive pressing and releasing of the nose ball is consecutively performed for 3 minutes or longer if nasal congestion is serious. The same is also performed on the opposite nostril. The above procedure was repeated once more where nasal congestion remains.

As for the administration to the nasopharynx, a syringe or spray with a nasopharyngeal injection nozzle, containing 10 ml of the composition is held such that the spray direction is the upward direction, with the head tilted forward about 45 degrees, and the nozzle is inserted deep so as to touch the nasopharynx wall, and then the composition is sprayed behind the throat and to the nasopharynx. The tip of the nozzle is slightly moved so as to apply the spray throughout the nasopharynx, so that the spray is applied to the entire surfaces of the back of the soft palate, the nasopharynx, and the retronasal route. Here, gargling was performed using the composition coming down in the mouth for 30 seconds. The administration to the nasopharynx was repeated once more.

As for the administration to the tonsils, 10 ml of the composition of the present invention is placed in a syringe or spray with a nasopharyngeal injection nozzle and then 10 ml of the composition is concentrated little by little on the painful inflammation site over one minutes or longer.

As for the administration to the trachea, bronchus, bronchioles, and lungs, a nozzle of a spray pump containing 10 ml of the composition is placed into the mouth in the upright position, and then lips are narrowed extremely and the inside of the neck is widened as much as possible. Thereafter, the composition is sprayed toward the throat while a strong breath is taken through the mouth. The spray enters the trachea, bronchus, bronchioles, and alveoli along the inhaled air.

Thereafter, the composition left after the treatments was placed in the mouth, and the chin was raised and then gargling was performed for 30 seconds while a slight breath was taken little by little.

The treatment for each site as described above was repeated once more within 1 hour after the first application was completed. As a result of investigating the degree of recovery in the cold or flu patients treated in these manners, symptoms, such as headache, runny nose, nose congestion, and fever, were eliminated or significantly relieved within 1-4 hours depending on the severity of infection, so that daily activities of the patients were not disturbed and recurrence or deterioration was prevented until several days later.

## Claims

1. A solid composition comprising an iodine agent and sodium chloride, wherein the solid composition comprises a mixture of 0.2-50 parts by weight of the iodine agent and 100 parts by weight of the sodium chloride and has a dissolution rate of 30 seconds or less in water.

2. The solid composition of claim 1, wherein the solid composition comprises a mixture of 0.4-25 parts by weight of the iodine agent and 100 parts by weight of the sodium chloride and has a dissolution rate of 15 seconds or less in water.

3. The solid composition of claim 1 or 2, wherein the iodine agent is selected from the group consisting of povidone iodine, cadexomer iodine, and poloxamer iodine.

4. The solid composition of claim 3, wherein the iodine agent is povidone iodine.

5. The solid composition of claim 1 or 2, wherein the solid composition is in a powder form and the size of powder particles is 30 mesh or more.

6. The solid composition of claim 1 or 2, wherein the mixture further comprises 0.001-2 parts by weight of a fragrant substance.

7. The solid composition of claim 6, wherein the fragrant substance includes at least one selected from butyl acetate, amyl acetate, isoamyl acetate, hexyl acetate, 2-hexyl acetate, ethyl propionate, butyl propionate, isobutyl propionate, isoamyl propionate, ethyl butyrate, ethyl 2-methylbutyrate, butyl isobutyrate, ethyl hexanoate, ethyl pivalate, methyl 4-methylvalerate, ethyl valerate, ethyl isovalerate, isopropyl isovalerate, ethyl lactate, 2-pentanone, heptanoic acid, 3-heptanone, 2,3-hexanedione, d-camphor, p-methyl anisole, 2-methoxy-6-methyl pyrazine, 1,8-cineole, 1,4-cineole, benzyl methyl ether, 3-heptanol, isoamyl isovalerate, butyl valerate, butyl lactate, methyl benzoate, ethyl benzoate, propyl benzoate, butyl benzoate, isobutyl benzoate, amyl benzoate, isoamyl benzoate, methyl phenylacetate, ethyl phenylacetate, benzyl acetate, sassafras acetate, isobornyl acetate, ethyl 3-phenylpropionate, 2-methyl-5-ethoxypyrazine, 2-methyl-6-ethoxypyrazine, 2,3-diethyl-5-methylpyrazine, 5-methyl-6,7-dihydro-5H-cyclopentapyrazine, 2-acetyl pyrazine, acetyl pyridine, 2-acetyl pyridine, 3-acetyl pyridine, 2-t-butyl cyclohexanone, p-dimethyl-hydroquinone, isoquinoline, 2,3-dimethyl-benzofuran, 3,6-dimethyl benzofuranone, ambernaphthofuran, p-t-butyl-cyclohexanone, thymol methyl ether, 3-phenylpropyl alcohol, 2,6-dimethoxyphenol, 2-t-butyl-6-methyl-phenol, 1-menthol, dl-menthol, d-neomenthol, menthone lactone, p-ethyl acetophenone, skatole, diphenyl ether, β-naphthyl methyl ether, β-naphthyl ethyl ether, saffron indenone, ambroxide, (-)-ambroxide, phenylacetic acid, watermelon ketone, dihydroactinidiolide, 3-butyl-phthalide, borneol, dl-isoborneol, thymol, exaltolide, exaltone, menthyl lactate, methyl dihydrojasmonate, ethylene brassylate, t-hydrofurfuryl phenylacetate, 2-(3-phenylpropyl)pyridine), (±)-muscone, (-)-muscone, isomuscone, normuscone, cyclohexadecanone, celestolide, sandal cyclopropane, aniseed oil, eucalyptus oil, peppermint oil, and spearmint oil.

8. An antiviral and antibacterial ocular, oral, nasal, or inhalation composition comprising, as an active ingredient, an aqueous solution obtained by dissolving the solid composition of any one of claims 1, 2, 4, and 7 in water, the aqueous solution containing 0.01-0.5 w/v% of an iodine agent and 1-5 w/v% of sodium chloride.

9. The antiviral and antibacterial ocular, oral, nasal, or inhalation composition of claim 8, wherein the iodine agent is selected from the group consisting of povidone iodine, cadexomer iodine, and poloxamer iodine.

10. The antiviral and antibacterial ocular, oral, nasal, or inhalation composition of claim 8, wherein the aqueous solution further contains 0.0001-0.01w/v% of the fragrant substance.

11. The antiviral and antibacterial ocular, oral, nasal, or inhalation composition of claim 10, wherein the fragrant substance is at least one fragrant substance selected from the group consisting of:
fragrant substances belonging to fragrant substance group A having a vapor pressure of 1.0-120 mmHg at normal temperature;
fragrant substances belonging to fragrant substance group B having a vapor pressure of 0.001-1.0 mmHg at normal temperature; and
fragrant substances belonging to fragrant substance group C having a vapor pressure of less than 0.001 mmHg at normal temperature.

12. The antiviral and antibacterial ocular, oral, nasal, or inhalation composition of claim 10, wherein the fragrant substance includes at least one selected from butyl acetate, amyl acetate, isoamyl acetate, hexyl acetate, 2-hexyl acetate, ethyl propionate, butyl propionate, isobutyl propionate, isoamyl propionate, ethyl butyrate, ethyl 2-methylbutyrate, butyl isobutyrate, ethyl hexanoate, ethyl pivalate, methyl 4-methylvalerate, ethyl valerate, ethyl isovalerate, isopropyl isovalerate, ethyl lactate, 2-pentanone, heptanoic acid, 3-heptanone, 2,3-hexanedione, d-camphor, p-methyl anisole, 2-methoxy-6-methyl pyrazine, 1,8-cineole, 1,4-cineole, benzyl methyl ether, 3-heptanol, isoamyl isovalerate, butyl valerate, butyl lactate, methyl benzoate, ethyl benzoate, propyl benzoate, butyl benzoate, isobutyl benzoate, amyl benzoate, isoamyl benzoate, methyl phenylacetate, ethyl phenylacetate, benzyl acetate, sassafras acetate, isobornyl acetate, ethyl 3-phenylpropionate, 2-methyl-5-ethoxypyrazine, 2-methyl-6-ethoxypyrazine, 2,3-diethyl-5-methylpyrazine, 5-methyl-6,7-dihydro-5H-cyclopentapyrazine, 2-acetyl pyrazine, acetyl pyridine, 2-acetyl pyridine, 3-acetyl pyridine, 2-t-butyl cyclohexanone, p-dimethyl-hydroquinone, isoquinoline, 2,3-dimethyl-benzofuran, 3,6-dimethyl benzofuranone, ambernaphthofuran, p-t-butyl-cyclohexanone, thymol methyl ether, 3-phenylpropyl alcohol, 2,6-dimethoxyphenol, 2-t-butyl-6-methyl-phenol, 1-menthol, dl-menthol, d-neomenthol, menthone lactone, p-ethyl acetophenone, skatole, diphenyl ether, β-naphthyl methyl ether, β-naphthyl ethyl ether, saffron indenone, ambroxide, (-)-ambroxide, phenylacetic acid, watermelon ketone, dihydroactinidiolide, 3-butyl-phthalide, borneol, dl-isoborneol, thymol, exaltolide, exaltone, menthyl lactate, methyl dihydrojasmonate, ethylene brassylate, t-hydrofurfuryl phenylacetate, 2-(3-phenylpropyl)pyridine), (±)-muscone, (-)-muscone, isomuscone, normuscone, cyclohexadecanone, celestolide, sandal cyclopropane, aniseed oil, eucalyptus oil, peppermint oil, and spearmint oil.

13. The antiviral and antibacterial ocular, oral, nasal, or inhalation composition of claim 11, wherein the fragrant substance is composed of:
at least one fragrant substance belonging to fragrant substance group A having a vapor pressure of 1.0-120 mmHg at normal temperature and being selected from butyl acetate, amyl acetate, isoamyl acetate, hexyl acetate, 2-hexyl acetate, ethyl propionate, butyl propionate, isobutyl propionate, isoamyl propionate, ethyl butyrate, ethyl 2-methylbutyrate, butyl isobutyrate, ethyl hexanoate, ethyl pivalate, methyl 4-methylvalerate, ethyl valerate, ethyl isovalerate, isopropyl isovalerate, ethyl lactate, 2-pentanone, heptanoic acid, 3-heptanone, 2,3-hexanedione, d-camphor, p-methyl anisole, 2-methoxy-6-methyl pyrazine, 1,8-cineole, 1,4-cineole, benzyl methyl ether, and 3-heptanol;
at least one fragrant substance belonging to fragrant substance group B having a vapor pressure of 0.001-1.0 mmHg at normal temperature and being selected from isoamyl isovalerate, butyl valerate, butyl lactate, methyl benzoate, ethyl benzoate, propyl benzoate, butyl benzoate, isobutyl benzoate, amyl benzoate, isoamyl benzoate, methyl phenylacetate, ethyl phenylacetate, benzyl acetate, sassafras acetate, isobornyl acetate, ethyl 3-phenylpropionate, 2-methyl-5-ethoxypyrazine, 2-methyl-6-ethoxypyrazine, 2,3-diethyl-5-methylpyrazine, 5-methyl-6,7-dihydro-5H-cyclopentapyrazine, 2-acetyl pyrazine, acetyl pyridine, 2-acetyl pyridine, 3-acetyl pyridine, 2-t-butyl cyclohexanone, p-dimethyl-hydroquinone, isoquinoline, 2,3-dimethyl-benzofuran, 3,6-dimethyl benzofuranone, ambernaphthofuran, p-t-butyl-cyclohexanone, thymol methyl ether, 3-phenylpropyl alcohol, 2,6-dimethoxyphenol, 2-t-butyl-6-methyl-phenol, 1-menthol, dl-menthol, d-neomenthol, menthone lactone, p-ethyl acetophenone, skatole, diphenyl ether, β-naphthyl methyl ether, β-naphthyl ethyl ether, saffron indenone, ambroxide, (-)-ambroxide, phenylacetic acid, watermelon ketone, dihydroactinidiolide, 3-butyl-phthalide, borneol, dl-isoborneol, thymol, aniseed oil, eucalyptus oil, peppermint oil, and spearmint oil; and
at least one fragrant substance belonging to fragrant substance group C having a vapor pressure of less than 0.001 mmHg at normal temperature and being selected from exaltolide, exaltone, menthyl lactate, methyl dihydrojasmonate, ethylene brassylate, t-hydrofurfuryl phenylacetate, 2-(3-phenylpropyl)pyridine, (±)-muscone, (-)-muscone, isomuscone, normuscone, cyclohexadecanone, celestolide, and sandal cyclopropane.

14. The antiviral and antibacterial ocular, oral, nasal, or inhalation composition of claim 13, wherein the fragrant substance belonging to fragrant substance group A having a vapor pressure of 1.0-120 mmHg at normal temperature is at least one selected from the group consisting of amyl acetate, isoamyl acetate, 2-hexyl acetate, ethyl propionate, butyl propionate, isobutyl propionate, ethyl butyrate, ethyl 2-methylbutyrate, butyl isobutyrate, methyl 4-methylvalerate, ethyl valerate, ethyl isovalerate, isopropyl isovalerate, 2-pentanone, 2,3-hexanedione, d-camphor, 1,8-cineole, and 1,4-cineole.

15. The antiviral and antibacterial ocular, oral, nasal, or inhalation composition of claim 13, wherein the fragrant substance belonging to fragrant substance group B having a vapor pressure of 0.001-1.0 mmHg at normal temperature is at least one selected from the group consisting of butyl valerate, ethyl benzoate, propyl benzoate, isobornyl acetate, isoquinoline, ambernaphthofuran, 1-menthol, d-neomenthol, diphenyl ether, β-naphthyl ethyl ether, ambroxide, and (-)-ambroxide.

16. The antiviral and antibacterial ocular, oral, nasal, or inhalation composition of claim 13, wherein the fragrant substance belonging to fragrant substance group C having a vapor pressure of less than 0.001 mmHg at normal temperature is selected from the group consisting of exaltolide, (±)-muscone, and (-)-muscone.

17. The antiviral and antibacterial ocular, oral, nasal, or inhalation composition of claim 10, wherein the antiviral and antibacterial composition comprises 0.01-0.5 w/v% of the iodine agent, 1.2-3.5 w/v% of the sodium chloride, and 0.0001-0.01 w/v% of the fragrant substance.

18. The antiviral and antibacterial ocular, oral, nasal, or inhalation composition of any one of claims 8 to 17, wherein the antiviral and antibacterial composition is used to prevent or treat a respiratory infectious disease.

19. The antiviral and antibacterial ocular, oral, nasal, or inhalation composition of claim 18, wherein the respiratory infectious disease is a viral infection.

20. The antiviral and antibacterial ocular, oral, nasal, or inhalation composition of claim 18 or 19, wherein the respiratory infectious disease is a cold or flu.

21. A method for prevention or treatment of a respiratory infectious disease by applying the antiviral and antibacterial composition of any one of claims 8 to 17 to at least one site selected from the group consisting of eyes, oral cavity, nasal cavity, paranasal sinuses, nasopharynx, oropharynx, laryngopharynx, tonsils, trachea, bronchus, bronchioles, and lungs.

22. The method of claim 21, wherein the site includes eyes, oral cavity, nasal cavity, paranasal sinuses, nasopharynx, oropharynx, laryngopharynx, tonsils, trachea, bronchus, bronchioles, and lungs.

23. The method of claim 22, wherein the antiviral and antibacterial composition is applied in a liquid form when the site is eyes, oral cavity, nasal cavity, and paranasal sinuses.

24. The method of claim 23, wherein when the site is nasal cavity, the antiviral and antibacterial composition is applied in a liquid form to the nasal cavity and then the nasal cavity is subjected to vibration, pulse, pressure variation, or liquid shaking.

25. The method of claim 22, wherein the antiviral and antibacterial composition is applied in a spray form when the site is nasopharynx, oropharynx, laryngopharynx, tonsils, trachea, bronchus, bronchioles, and lungs.
